# EUROPEAN PATENT SPECIFICATION

(11) **EP 3 194 483 B1**
(45) Date of publication and mention of the grant of the patent: **19.12.2018**
(21) Application number: 15780974.0
(22) Date of filing: 15.09.2015
(51) Int. Cl.: B05D 1/02, C07C 69/003, C07C 69/75, C07C 69/78, C07C 63/06, C08K 5/00, C08K 5/103, F16F 15/02, C08L 27/00

(54) **POLYMERIC COMPOSITIONS WITH IMPROVED NOISE SUPPRESSION**
POLYMERZUSAMMENSETZUNGEN MIT VERBESSERTER GERÄUSCHUNTERDRÜCKUNG
COMPOSITIONS POLYMÈRES PRÉSENTANT UNE SUPPRESSION AMÉLIORÉE DU BRUIT

(30) Priority: 16.09.2014 US 201462050942 P; 14.09.2015 US 201514852662
(43) Date of publication of application: 26.07.2017
(73) Proprietor: Eastman Chemical Company, Kingsport, TN 37660 (US)
(72) Inventor: FUNDERBURG, Michael Dean, Jr., Gray, TN 37615 (US); DELOACH, Joseph Alexander, Jonesborough, TN 37659 (US); MORALEZ, Jesus Gabriel, Kingsport, TN 37664 (US); BAKER, John Dayton, Jr., Kingsport, Tennessee 37663-0273 (US); BRAMMER, Larry E., Jr., Kingsport, TN 37660 (US); CLUBB, Clyde Neal, Longview, TX 75605 (US); COOK, Steven Leroy, Kingsport, TN 37664 (US); ZHENG, Pinguan, Johnson City, TN 37601 (US)
(74) Representative: Ricker, Mathias
(86) International application number: PCT/US2015/050125
(87) International publication number: WO 2016/044230

(56) References cited:
- EP-A2- 0 416 822
- WO-A2-2008/011536
- US-A- 4 584 215
- US-A1- 2001 044 486
- US-A1- 2007 037 926

## Description

### CROSS-REFERENCE TO RELATED APPLICATION

This application claims priority to U. S. Provisional Application Number 62/050942 filed September 16, 2014.

### FIELD OF THE INVENTION

The invention relates to improving vibration damping on a substrate. More specifically, the invention relates to a method of improve vibration damping on a substrate, such as the underbody of a vehicle, by using plastisols.

### BACKGROUND OF THE INVENTION

The objective of this invention is to provide improved vibration damping performance to metallic substrates. Examples of such substrates include, but are not limited to, those used for the construction of vehicles. More specifically, the objective of this invention is to provide improved vibration damping within the range of temperatures frequently encountered during driving, namely from -30° to 50°C and most frequently from -10°C to 40°C. Another objective of this invention is to provide improved vibration damping within this temperature range across the frequencies audible to humans, particularly in the low frequency range of 10 to 200 Hz as described in "Low Frequency Noise. What we know, what we do not know, and what we would like to know", Leventhall, Geoff, Journal of Low Frequency Noise, Vibration and Active Control 28, 2, pp. 79-104 (2009).

The reduction of noise, vibration, and harshness (often abbreviated as NVH) to humans is a goal of many industrial processes. Exposure to NVH comes from numerous sources, and can be mitigated by various means. For example, laminated safety glass can be comprised of acoustic interlayers which suppress sound transmission. Applications of such acoustic interlayers can include glass panes in commercial and residential buildings and automotive glazing. Other sources of NVH in vehicles include engine noise, road noise, springs and suspensions, braking, and chassis vibration. Noise suppression techniques include component design to reduce vibration and sound transmission; use of composite materials instead of metals; elastomeric sleeves or guards; nonwoven fabrics; carpet or other materials applied to the vehicle interior; foam; liquid-applied damping formulations; and objects produced from viscoelastic materials, such as bitumen or asphaltic pads. Although effective to varying extents depending on the source of the noise, these techniques suffer from limitations. For example, asphaltic pads cannot easily be placed and conformed to some locations on a vehicle body, require manual application, are subject to embrittlement, and must continue to adhere to the metal substrate in order to be effective. Some materials contribute undesired weight to the vehicle, contrary to weight reduction goals designed to improve fuel mileage. Materials which require high temperature and/or long times to cure can slow production, add cost, and result in higher energy usage.

One mode of NVH is through vibration. Polymeric materials can damp, or reduce oscillations of, a substrate by dissipating the oscillation energy with their viscoelastic behavior. A standard measurement of damping utilizes the Oberst method and apparatus. In this method, a material engineered to confer damping behavior is affixed to a stainless steel bar which has negligible damping itself. The effect of the damping material is deduced from the behavior of the sample bar compared to an untreated reference bar. Damping behavior may also be measured using Dynamic Mechanical Thermal Analysis, or DMTA. In this technique, a sample is exposed to a sinusoidal force, generally over a range of temperatures or frequencies. When heated, the modulus of a viscoelastic polymeric substance varies greatly from the glassy state at low temperatures, through the glass transition to a rubbery state, and finally to a lower viscosity molten state. The ratio of the storage modulus to the loss modulus, a value known as the tan δ, is a measure of the material's ability to damp vibrations. Higher tan δ values signify more effective damping behavior. The DMTA tan δ has been shown to correlate well with the Oberst bar testing.

Plasticized polyvinyl chloride (PVC) is well known in the automotive industry. Plasticized PVC applied as a plastisol in automotive underbody coatings and sealants, after thermal curing, can protect the vehicle from chipping by stones and other materials on the road surface. Such coatings also offer protection against corrosion, for example from salted roads. Plasticized PVC coatings can also provide a low level of reduction of the transmission of vibrations from metallic substrates. However, the performance of plasticized PVC coatings is inadequate to confer satisfactory vibration damping across the range of temperatures and noise frequencies typically encountered without the incorporation of additional damping techniques. These performance deficiencies are exacerbated when the desire to reduce NVH to vehicle passengers over traditional levels is considered. Despite these deficiencies, the ease of application and economy of PVC plastisols make them an appealing potential solution to the reduction of NVH should performance improvements be realized.

### SUMMARY OF THE INVENTION

The present invention relates to a method of improving vibration damping of a substrate comprising affixing a plastisol to the substrate, wherein the plastisol comprises a polymeric component and a plasticizer. The plasticizer comprises the esterification product formed by the reaction of formula (I) 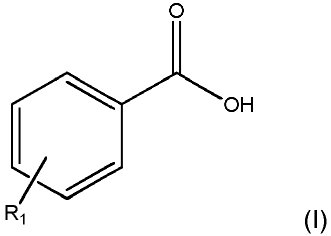 and formula (II) 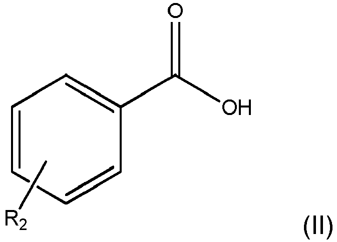 or the corresponding esters, anhydrides, and/or acid chlorides of formula (I) and/or formula (II), with formula (III) 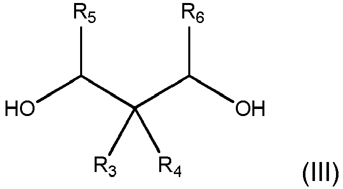 R₁ and R₂ are independently selected from the group consisting of hydrogen and linear or branched alkyl groups containing 1 to 9 carbon atoms. R₃ and R₄ are independently selected from the group consisting of hydrogen and linear or branched alkyl groups having 1 to 8 carbon atoms. The sum of the number of carbon atoms of R₃ and R₄ ranges from 0 to 10. R₅ and R₆ are independently selected from the group consisting of hydrogen and linear or branched alkyl groups having 1 to 8 carbon atoms. The sum of the number of carbon atoms of R₅ and R₆ ranges from 0 to 10.

### DETAILED DESCRIPTION OF THE INVENTION

As used herein the term "and/or," when used in a list of two or more items, means that any one of the listed items can be employed by itself, or any combination of two or more of the listed items can be employed. For example, if a composition is described as containing components A, B, and/or C, the composition can contain A alone; B alone; C alone; A and B in combination; A and C in combination; B and C in combination; or A, B, and C in combination.

The term "affixing", as used herein, refers to providing continuous and intimate contact between the plastisol and the substrate such that the fused plastisol remains on the substrate. For example, a plastisol can be affixed to a car underbody via spray coating the plastisol onto a car underbody and subjecting the coated car underbody to conditions to fuse the plastisol. The term "adhering" as used herein, refers to using an adhesive to affix a fused plastisol sheet to a substrate.

The term "esterification product", as used herein, refers to the blend of "partial esters", "mixed esters", and "diesters" produced from the reaction of one or more carboxylic acids with a diol. The term "partial esters", as used herein, refers to the reaction product wherein not all of the hydroxyls of a diol have fully reacted with a carboxylic acid. The term "mixed ester", as used herein, refers to the reaction product wherein each of the hydroxyls of a diol has reacted with different carboxylic acids. The term "diester", as used herein, refers to the reaction product wherein each hydroxyl of a diol has reacted with the same carboxylic acid. For example, if benzoic acid and toluic acid are reacted with 1-3-pentanediol, the esterification product can comprise the partial esters 1,3-pentanediol monobenzoate and 1,3-pentanediol monotoluate, the mixed ester 1,3-pentanediol benzoate/toluate, and the diesters 1,3-pentanediol dibenzoate and 1,3-pentanediol ditoluate. The term "the reaction product of formula (a) and/or formula (b) with formula (c)", as used herein, is intended to include carboxylic acids and the the corresponding esters, anhydrides, and/or acid chlorides of formula (a) and/or formula (b) as explicitly set forth in the claims.

The term "plastisol", as used herein, refers to a liquid dispersion of polymeric resin particles, optionally with other ingredients, in a plasticizer. The term "fused plastisol", as used herein, refers to the solid plastic material that is formed upon fusing the plastisol and subsequently cooling to a desired temperature. The term "fusing", as used herein, refers to heating of the plastisol to a temperature sufficient to yield a solid structure with mechanical integrity.

The term "substrate", as used herein, refers to the material that provides the surface onto which the plastisol is affixed.

Disclosed herein is a plasticizer comprising the esterification product formed by the reaction of formula (I) 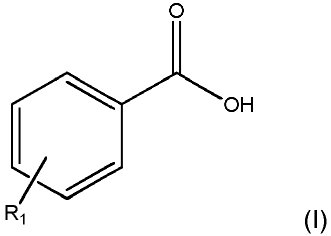 and formula (II) 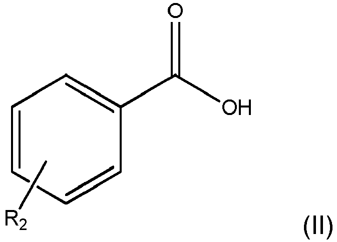 or the corresponding esters, anhydrides, and/or acid chlorides of formula (I) and/or formula (II), with formula (III) 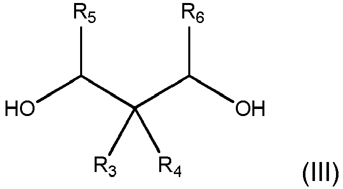 R₁ and R₂ are independently selected from the group consisting of hydrogen and linear or branched alkyl groups containing 1 to 9 carbon atoms, with the proviso that R₁ and R₂ are not each hydrogen. R₃ and R₄ are independently selected from the group consisting of hydrogen and linear or branched alkyl groups having 1 to 8 carbon atoms. The sum of the number of carbon atoms of R₃ and R₄ ranges from 0 to 10. R₅ and R₆ are independently selected from the group consisting of hydrogen and linear or branched alkyl groups having 1 to 8 carbon atoms. The sum of the number of carbon atoms of R₅ and R₆ ranges from 0 to 10.

In one aspect, R₁ and R₂ are independently hydrogen or methyl, with the proviso that R₁ and R₂ are not each hydrogen. R₃, R₄, R₅, and R₆ can independently be hydrogen or linear or branched alkyl groups having 1 to 4 carbon atoms. In one aspect the plasticizer comprises 2,2,4-trimethyl-1,3-pentanediol ditoluate, 2,2,4-trimethyl-1,3-pentanediol benzoate/toluate, 2,4-dimethyl-2-ethyl-1,3-hexanediol ditoluate, 2,4-dimethyl-2-ethyl-1,3-hexanediol benzoate/toluate, 2-methyl-2-propyl-1,3-propanediol ditoluate, and/or 2-methyl-2-propyl-1,3-propanediol benzoate/toluate.

In one aspect, the plasticizer comprises 2,2,4-trimethyl-1,3-pentanediol ditoluate and/or 2,2,4-trimethyl-1,3-pentanediol benzoate/toluate. In one aspect, the plasticizer comprises 2,4-dimethyl-2-ethyl-1,3-hexanediol dibenzoate.

One skilled in the art can readily make the above-identified plasticizers using process conditions well known in the art. For example, the proportions of mixed esters formed when two carboxylic acids are used may be adjusted by varying the ratios of the two carboxylic acids.

The esterification product formed by the reactions of the carboxylic acids of formula (I) and formula (II) with the diol of formula (III) can contain several reaction products including (1) partial esters formed when not all of the of the hydroxyls of formula (III) are fully reacted with either formula (I) or formula (II), (2) mixed esters formed by the reaction of formula (I) with one hydroxyl of formula (III) and formula (II) with the other hydroxyl of formula (III) when R₁ and R₂ are different, and (3) diesters formed by the reaction of either formula (I) or formula (II) with both of the hydroxyls of formula (III).

In one aspect, the plasticizer comprises the esterification product formula (IV) 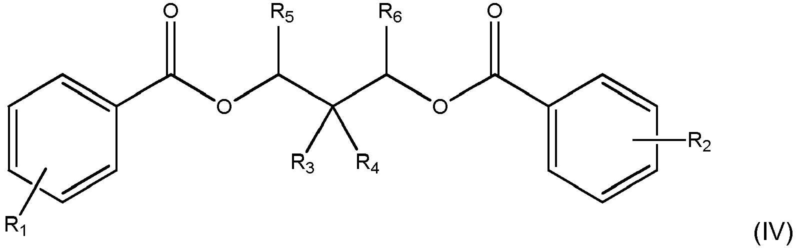 The esterification product comprises at least 10 weight percent of a mixed ester based on the total weight of the esterification product. Other examples of the amount of mixed ester based on the total weight of the esterification product include at least 15 weight percent, at least 20 weight percent, at least 25 weight percent, at least 30 weight percent, at least 40 weight percent, or at least 50 weight percent of a mixed ester. In one aspect, the amount of mixed ester ranges from 10 weight percent to 80 weight percent, 10 weight percent to 50 weight percent, 10 weight percent to 40 weight percent, 20 weight percent to 80 weight percent, 20 weight percent to 50 weight percent, or 20 weight percent to 50 weight percent, based on the total weight of the esterification product.

Also disclosed herein is a plastisol comprising a polymeric component and a plasticizer. The plasticizer comprises the esterification product formed by the reaction of formula (I) 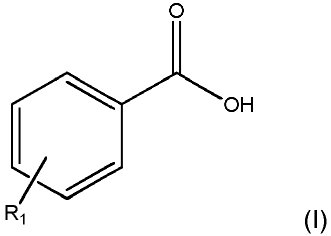 and formula (II) 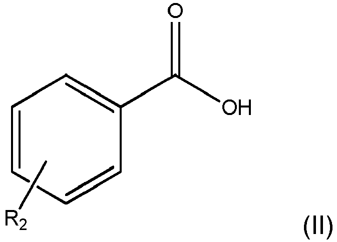 or the corresponding esters, anhydrides, and/or acid chlorides of formula (I) and/or formula (II), with formula (III) 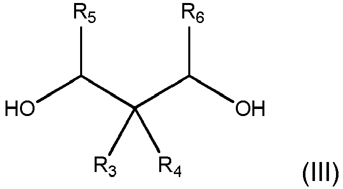 R₁ and R₂ are independently selected from the group consisting of hydrogen and linear or branched alkyl groups containing 1 to 9 carbon atoms. R₃ and R₄ are independently selected from the group consisting of hydrogen and linear or branched alkyl groups having 1 to 8 carbon atoms. The sum of the number of carbon atoms of R₃ and R₄ ranges from 0 to 10. R₅ and R₆ are independently selected from the group consisting of hydrogen and linear or branched alkyl groups having 1 to 8 carbon atoms. The sum of the number of carbon atoms of R₅ and R₆ ranges from 0 to 10.

In one aspect, R₁ and R₂ are independently selected from the group consisting of hydrogen and methyl. R₃ and R₄ are independently selected from the group consisting of hydrogen and linear or branched alkyl groups having 1 to 4 carbon atoms and R₅ and R₆ are independently selected from the group consisting of hydrogen and linear or branched alkyl groups having 1 to 4 carbon atoms. In another aspect, R₁ and R₂ are not each hydrogen.

In another aspect, the plasticizer comprises 2,2,4-trimethyl-1,3-pentanediol ditoluate, 2,2,4-trimethyl-1,3-pentanediol benzoate/toluate, 2,2,4-trimethyl-1,3-pentanediol dibenzoate, 2,4-dimethyl-2-ethyl-1,3-hexanediol ditoluate, 2,4-dimethyl-2-ethyl-1,3-hexanediol benzoate/toluate, 2,4-dimethyl-2-ethyl-1,3-hexanediol dibenzoate, 2-methyl-2-propyl-1,3-propanediol ditoluate, 2-methyl-2-propyl-1,3-propanediol benzoate/toluate, 2-methyl-2-propyl-1,3-propanediol dibenzoate, 2-butyl-2-ethyl-1,3-propanediol dibenzoate, 2-butyl-2-ethyl-1,3-propanediol ditoluate, and/or 2-butyl-2-ethyl-1,3-propanediol benzoate/toluate. In one aspect, the plasticizer comprises 2,2,4-trimethyl-1,3-pentanediol ditoluate, 2,2,4-trimethyl-1,3-pentanediol dibenzoate, 2,4-dimethyl-2-ethyl-1,3-hexanediol ditoluate, and/or 2,4-dimethyl-2-ethyl-1,3-hexanediol dibenzoate. In one aspect, the plasticizer comprises 2-butyl-2-ethyl-1,3-propanediol dibenzoate, 2-butyl-2-ethyl-1,3-propanediol ditoluate, and/or 2-butyl-2-ethyl-1,3-propanediol benzoate/toluate.

In one aspect, the plasticizer comprises the esterification product (IV) 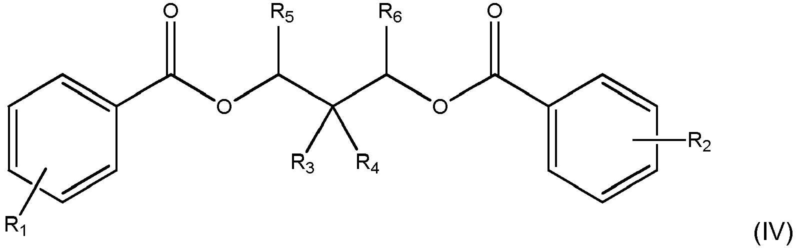 wherein the esterification product comprises at least 10 weight percent of a mixed ester, based on the total weight of the esterification product. Other examples of the amount of mixed ester based on the total weight of the esterification product include at least 15 weight percent, at least 20 weight percent, at least 25 weight percent, at least 30 weight percent, at least 40 weight percent or at least 50 weight percent of a mixed ester. In one aspect, the amount of mixed ester ranges from 10 weight percent to 80 weight percent, 10 weight percent to 50 weight percent, 10 weight percent to 40 weight percent, 20 weight percent to 80 weight percent, 20 weight percent to 50 weight percent, or 20 weight percent to 40 weight percent, based on the total weight of the esterification product

In addition to the plasticizer, the plastisol comprises a polymeric component. In one aspect, the polymeric component comprises polyvinyl chloride, polyvinyl acetate, an acrylic polymer, and/or a vinyl chloride-containing copolymers. In one aspect, the polymeric component comprises polyvinyl chloride and/or an acrylic polymer. In one aspect, the polymeric component comprises polyvinyl chloride and/or polyvinyl acetate. In one aspect, the polymeric component comprises polyvinyl chloride and/or vinyl chloride-containing copolymers comprising vinyl acetate. In one aspect, the polymeric component comprises polyvinyl chloride and vinyl chloride-containing copolymers comprising acrylic. In one aspect, the polymeric component comprises polyvinyl chloride.

The plastisol comprises plasticizer, polymeric component, and other components. Examples of other components include, but are not limited to, a second plasticizer, fillers, pigments, stabilizers, foaming agents, hollow materials, elastomeric materials, rheology control additives, and adhesion promoters. The amounts of plasticizer, polymeric component, and other components can vary widely. For example, in one aspect the plastisol comprises 10 weight percent to 70 weight percent plasticizer, 10 weight percent to 70 weight percent polymeric component, and 10 weight percent to 80 weight percent other components, each based on the total weight of the plastisol, Other examples include, 15 weight percent to 60 weight percent plasticizer, 15 weight percent to 60 weight percent polymeric component, and 10 weight percent to 60 weight percent other components; or 20 weight percent to 45 weight percent plasticizer, 20 weight percent to 45 weight percent polymeric component, and 10 weight percent to 50 weight percent other components.

The viscosity of the plastisol can vary over a wide range. In one aspect, the plastisol has a viscosity ranging from 5,000 centipoise (cP) to 200,000 cP using Brookfield viscosity measurement at 23 °C. In other examples, the plastisol has a viscosity ranging from 30,000 cP to 120,000 cP or from 40,000 cP to 90,000 cP.

In one aspect, the plastisol comprises a second plasticizer. In one aspect the second plasticizer comprises phthalates; terephthalates; isophthalates; trimellitates; adipates; cyclohexanedicarboxylates; benzoates; phosphates; diesters of ethylene glycol, propylene glycol, their oligomers, and mixtures thereof; citrates; succinates; alkyl sulfonates; fatty acid esters and epoxidized fatty acid esters; triglycerides and epoxidized triglycerides, optionally substituted; dianhydrohexitol diesters; pentaerythritol-based tetraesters; furan-based esters; other esters; ketals; and/or polymeric plasticizers. In another aspect, the second plasticizer comprises dioctyl terephthalate, diisooctyl phthalate, di-2-ethylhexyl phthalate, di-2-ethylhexyl terephthalate, tri-2-ethylhexyl trimellitate, di-2-propylheptyl phthalate, diisononyl phthalate, diisodecyl phthalate, diisoundecyl phthalate, ditridecyl phthalate, trioctyl trimellitate, triisononyl trimellitate, 2,2,4-trimethyl-1,3-pentanediol diisobutyrate, isononyl benzoate, isodecyl benzoate, diisononyl 1,2-cyclohexanedicarboxylate, dioctyl adipate, di-2-ethylhexyl adipate, triethylene glycol di-2-ethylhexanoate, diethylene glycol dibenzoate, dipropylene glycol dibenzoate, and/or dibenzoates produced from mixtures of diethylene glycol and dipropylene glycol. In one aspect, the second plasticizer comprises dioctyl terephthalate, di-2-ethylhexyl terephthalate, dioctyl adipate, di-2-ethylhexyl adipate, and/or triethylene glycol di-2-ethylhexanoate. In one aspect, the second plasticizer comprises, di-2-ethylhexyl terephthalate, diisononyl phthalate, and/or diisononyl 1,2-cyclohexanedicarboxylate.

In one aspect, the plastisol comprises fillers. Nonlimiting examples of fillers include calcium carbonate, magnesium carbonate, silica, clay, mica, graphite, zinc oxide, and/or calcium oxide. In one aspect, the fillers comprise calcium carbonate.

The plastisol, in one aspect, comprises stabilizers. Nonlimiting examples of stabilizers include metal soaps, epoxidized oils and epoxidized fatty acid esters, and/or organotin compounds.

In one aspect, the plastisol can be formulated or produced in a manner which incorporates more free volume into the fused plastisol. In one such technique, mechanical frothing may be applied to produce a foamed plastisol. In another aspect, a chemical foaming agent which results in a foamed structure after fusing is completed. One non-limiting example of such a foaming agent is azodicarbonamide. In one aspect, a catalyst is used along with the chemical foaming agent. In another aspect, foam stabilizers are used. In another aspect, hollow materials are incorporated into the plastisol. Nonlimiting examples of hollow materials include glass beads, microbeads, and/or microspheres, which can be produced from either inorganic or polymeric organic substances. In one aspect, the hollow materials are thermoplastic microspheres.

In one aspect, the plastisol comprises elastomeric materials. Nonlimiting examples of elastomeric materials include nitrile-butadiene rubber, natural rubber, isoprene rubber, butadiene rubber, styrene-butadiene rubber, butyl rubber, ethylene-propylene-diene monomer (EPDM) rubber, chloroprene rubber, styrenated block copolymers, ethylene-vinyl acetate copolymers, olefinic elastomers, olefinic copolymer elastomers, silicone elastomers, polysulfide elastomers, and/or polyurethane elastomers.

In another aspect, additives to control rheology can be incorporated into the plastisols. These may include secondary plasticizers or diluents. Examples of such additives include petroleum distillates; hydrocarbon oils such as, for example, mineral oil and mineral spirits; fatty acid esters; polyphenyl oligomers, optionally partially hydrogenated; and organic solvents. Conversely, thickeners may be added to boost viscosity as desired. Materials and techniques for adjusting plastisol rheology are well known in the art.

In one aspect, the plastisol comprises adhesion promoters. Nonlimiting examples of adhesion promoters include polyamidoamines, blocked isocyanates and isocyanurates, silanes, and/or epoxy resins.

in one aspect, the fused plastisol has a maximum Tan Delta (Tan δₘₐₓ) occurring between 20 °C and 50 °C and has Tan Delta at 30 °C (Tan δ_{30C}) ranging from 0.5 to 2.0, when measured on a sample nominally 0.6-0.7 mm thick, 3.2 mm wide, and 10-12 mm long using a Q800 Dynamic Mechanical Analyzer with a Tension Clamp at a strain of 0.1% and at a frequency of 1 Hz and a temperature ramp rate of 3 °C/min.

In one aspect, Tan Delta at 30 °C (Tan δ_{30C}) ranges fom 0.5 to 1.8 or 0.5 to 1.6 or 0.5 to 1.4 or 0.6 to 2.0 or 0.6 to 1.8 or 0.6 to 1.6 or 0.6 to 1.4 or 0.7 to 2.0 or 0.7 to 1.8 or 0.7 to 1.6 or 0.7 to 1.4. In one aspect, the maximum Tan Delta (Tan δₘₐₓ) occurs between 20 °C and 40 °C or 30 °C and 50 °C

The present invention relates to a method of improving vibration damping of a substrate comprising affixing a plastisol to the substrate, wherein the plastisol comprises a polymeric component and a plasticizer. The plasticizer comprises the esterification product formed by the reaction of formula (I) 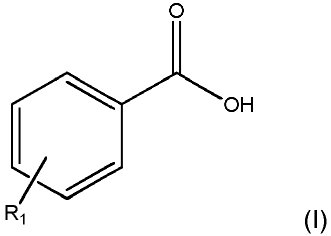 and formula (II) 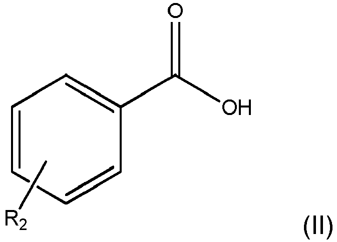 or the corresponding esters, anhydrides, and/or acid chlorides of formula (I) and/or formula (II), with formula (III) 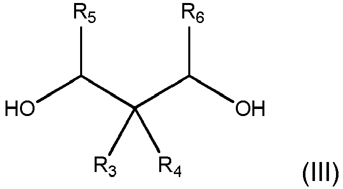 R₁ and R₂ are independently selected from the group consisting of hydrogen and linear or branched alkyl groups containing 1 to 9 carbon atoms. R₃ and R₄ are independently selected from the group consisting of hydrogen and linear or branched alkyl groups having 1 to 8 carbon atoms. The sum of the number of carbon atoms of R₃ and R₄ ranges from 0 to 10. R₅ and R₆ are independently selected from the group consisting of hydrogen and linear or branched alkyl groups having 1 to 8 carbon atoms. The sum of the number of carbon atoms of R₅ and R₆ ranges from 0 to 10.

All of the aspects of the plastisol described herein above can apply to the method of improving vibration damping of a substrate. These aspects include the options for R₁, R₂, R₃, R₄, R₅, and R₆ groups, plasticizer, the amount of mixed esters, polymeric component, the amounts of plasticizer, polymeric component, and other components in the plastisol, plastisol viscosity ranges, second plasticizers, fillers, stabilizers, foaming agents, hollow materials, elastomeric materials, rheology control additives, adhesion promoters, maximum Tan Delta and Tan Delta at 30 °C.

The substrate is not particularly limited. In one aspect, the substrate is metal. In one aspect, the substrate comprises steel. In one aspect, the substrate comprises aluminum. In one aspect, the substrate is part of a wheeled vehicle. In another aspect, the substrate is on the underbody of a wheeled vehicle.

In one aspect, the method of affixing the plastisol onto the substrate comprises (a) applying the plastisol onto the substrate, (b) fusing the plastisol to produce a plastisol-covered substrate, and (c) cooling the plastisol-covered substrate to ambient temperatures. The method for applying the plastisol onto the substrate is not particularly limited. In one aspect, applying the plastisol onto the substrate comprises coating the substrate with the plastisol. Nonlimiting examples of coating include spray coating and/or extrusion coating.

In one aspect, the method of affixing the plastisol to the substrate comprises (a) fusing the plastisol into a sheet and (b) adhering the sheet to the substrate.

In one aspect, the fusing occurs at a temperature ranging from 100 °C to 220 °C for a time period ranging from 1 min to 2 hours. In another aspect, the fusing occurs at a temperature ranging from 140 °C to 180 °C for a time period ranging from 15 min. to 40 min.

Listed below are non-limiting embodiments of the present disclosure and invention.
A1. A plasticizer comprising the esterification product formed by the reaction of formula (I) 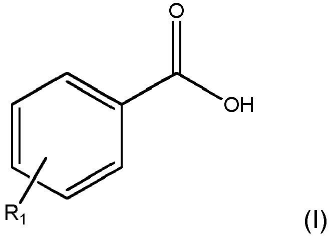 and formula (II) 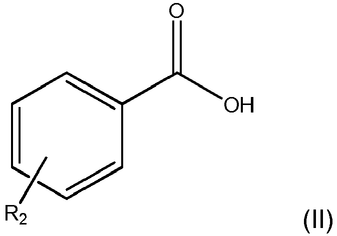 or the corresponding esters, anhydrides, and/or acid chlorides of formula (I) and/or formula (II), with formula (III) 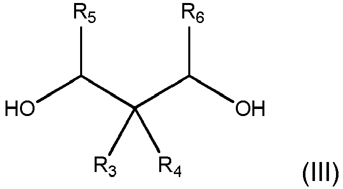 wherein R₁ and R₂ are independently selected from the group consisting of hydrogen and linear or branched alkyl groups containing 1 to 9 carbon atoms, with the proviso that R₁ and R₂ are not each hydrogen; R₃ and R₄ are independently selected from the group consisting of hydrogen and linear or branched alkyl groups having 1 to 8 carbon atoms whereby the sum of the number of carbon atoms of R₃ and R₄ ranges from 0 to 10; and R₅ and R₆ are independently selected from the group consisting of hydrogen and linear or branched alkyl groups having 1 to 8 carbon atoms whereby the sum of the number of carbon atoms of R₅ and R₆ ranges from 0 to 10.
A2. The plasticizer according to embodiment A1, wherein R₁ and R₂ are independently hydrogen or methyl, with the proviso that R₁ and R₂ are not each hydrogen; R₃ and R₄ are independently selected from the group consisting of hydrogen and linear or branched alkyl groups having 1 to 4 carbon atoms and R₅ and R₆ are independently selected from the group consisting of hydrogen and linear or branched alkyl groups having 1 to 4 carbon atoms.
A3. The plasticizer according to any of embodiments A1-A2, wherein the plasticizer comprises 2,2,4-trimethyl-1,3-pentanediol ditoluate, 2,2,4-trimethyl-1,3-pentanediol benzoate/toluate, 2,4-dimethyl-2-ethyl-1,3-hexanediol ditoluate, 2,4-dimethyl-2-ethyl-1,3-hexanediol benzoate/toluate, 2-methyl-2-propyl-1,3-propanediol ditoluate, 2-methyl-2-propyl-1,3-propanediol benzoate/toluate, or 2-butyl-2-ethyl-1,3-propanediol dibenzoate
A4. The plasticizer according to any of embodiments A1-A3, comprising the esterification product formula (IV) 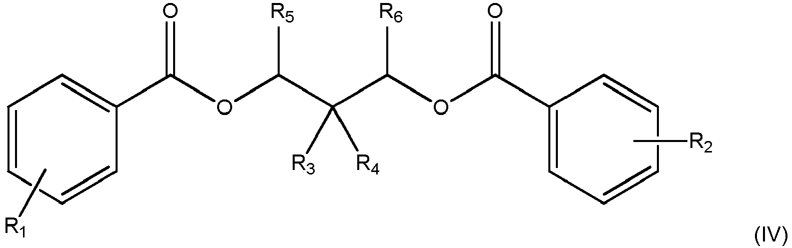 and wherein the esterification product comprises at least 10 weight percent of a mixed ester, based on the total weight of the esterification product; comprises at least 15 weight percent, at least 20 weight percent, 25 weight percent, at least 30 weight percent, at least 40 weight percent or at least 50 weight percent of a mixed ester.
A5. The plasticizer of embodiment A1, comprising 2,4-dimethyl-2-ethyl-1,3-hexanediol dibenzoate.
B1. A plastisol comprising a polymeric component and a plasticizer wherein the plasticizer comprises the esterification product formed by the reaction of formula (I) 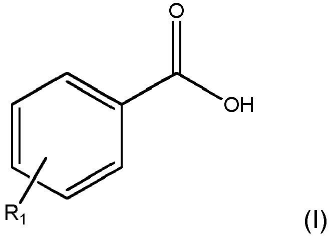 and formula (II) 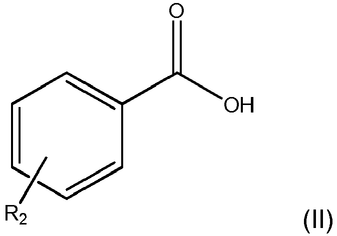 or the corresponding esters, anhydrides, and/or acid chlorides of formula (I) and/or formula (II), with formula (III) 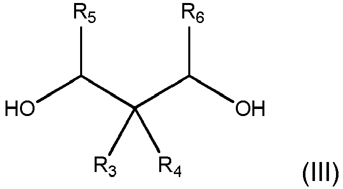 wherein R₁ and R₂ are independently selected from the group consisting of hydrogen and linear or branched alkyl groups containing 1 to 9 carbon atoms; R₃ and R₄ are independently selected from the group consisting of hydrogen and linear or branched alkyl groups having 1 to 8 carbon atoms whereby the sum of the number of carbon atoms of R₃ and R₄ ranges from 0 to 10; and R₅ and R₆ are independently selected from the group consisting of hydrogen and linear or branched alkyl groups having 1 to 8 carbon atoms whereby the sum of the number of carbon atoms of R₅ and R₆ ranges from 0 to 10.
B2. The plastisol according to embodiment B1, wherein R₁ and R₂ are independently selected from the group consisting of hydrogen and methyl; R₃ and R₄ are independently selected from the group consisting of hydrogen and linear or branched alkyl groups having 1 to 4 carbon atoms and R₅ and R₆ are independently selected from the group consisting of hydrogen and linear or branched alkyl groups having 1 to 4 carbon atoms.
B3. The plastisol according to any of embodiments B1-B2, with the proviso that R₁ and R₂ are not each hydrogen.
B4. The plastisol according to any of embodiments B1-B3, wherein the plasticizer comprises 2,2,4-trimethyl-1,3-pentanediol ditoluate, 2,2,4-trimethyl-1,3-pentanediol benzoate/toluate, 2,2,4-trimethyl-1,3-pentanediol dibenzoate, 2,4-dimethyl-2-ethyl-1,3-hexanediol ditoluate, 2,4-dimethyl-2-ethyl-1,3-hexanediol benzoate/toluate, 2,4-dimethyl-2-ethyl-1,3-hexanediol dibenzoate, 2-methyl-2-propyl-1,3-propanediol ditoluate, 2-methyl-2-propyl-1,3-propanediol benzoate/toluate, 2-methyl-2-propyl-1,3-propanediol dibenzoate, 2-butyl-2-ethyl-1,3-propanediol dibenzoate, 2-butyl-2-ethyl-1,3-propanediol ditoluate, 2-butyl-2-ethyl-1,3-propanediol benzoate/toluate, or 2-butyl-2-ethyl-1,3-propanediol dibenzoate.
B5. The plastisol according to any of embodiments B1-B4, wherein the plasticizer comprises 2,2,4-trimethyl-1,3-pentanediol ditoluate, 2,2,4-trimethyl-1,3-pentanediol dibenzoate, 2,4-dimethyl-2-ethyl-1,3-hexanediol ditoluate, 2,4-dimethyl-2-ethyl-1,3-hexanediol dibenzoate, or 2-butyl-2-ethyl-1,3-propanediol dibenzoate.
B6. The plastisol according to any of embodiments B1-B5, wherein the plasticizer comprises the esterification product formula (IV) 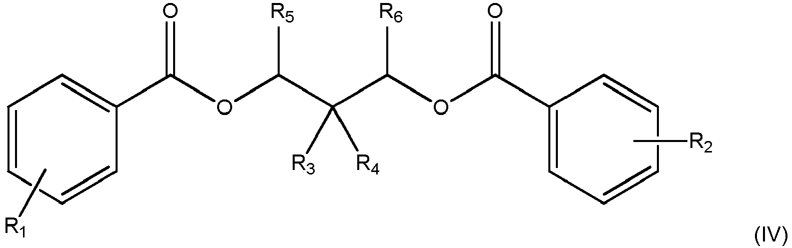 and wherein the esterification product comprises at least 10 weight percent of a mixed ester, based on the total weight of the esterification product; wherein the esterification product comprises at least 15 weight percent, at least 20 weight percent, at least 25 weight percent, at least 30 weight percent, at least 40 weight percent, or at least 50 weight percent of a mixed ester.
B7. The plastisol according to any of embodiments B1-B6, comprising 10 weight percent to 70 weight percent of the plasticizer, 10 weight percent to 70 weight percent of the polymeric component, and 10 weight percent to 80 weight percent of other components, each based on the total weight of the plastisol, and wherein the other components comprise a second plasticizer, fillers, pigments, stabilizers, foaming agents, hollow materials, elastomeric materials, rheology control additives, or adhesion promoters; or 15 weight percent to 60 weight percent of the plasticizer, 15 weight percent to 60 weight percent of the polymeric component, and 10 weight percent to 60 weight percent of other components; or 20 weight percent to 45 weight percent of the plasticizer, 20 weight percent to 45 weight percent of the polymeric component, and 10 weight percent to 50 weight percent of other components.
B8. The plastisol according to any of embodiments B1-B7, wherein the polymeric component comprises polyvinyl chloride, polyvinyl acetate, acrylic polymers, and/or vinyl chloride-containing copolymers; or wherein the polymeric component comprises polyvinyl chloride.
B9. The plastisol according to embodiment B8, wherein the polymeric component comprises the polyvinyl chloride and the acrylic polymer; the polyvinyl chloride and the polyvinyl acetate; the polyvinyl chloride and the vinyl chloride-containing copolymers comprising acetate; or the polyvinyl chloride and the vinyl chloride-containing copolymers comprising acrylic.
B10. The plastisol according to any of embodiments B1-B9, wherein the plastisol has a viscosity ranging from 5,000 to 200,000 cP using Brookfield viscosity measurement at 23 °C; wherein the plastisol has a viscosity ranging from 30,000 to 120,000 cP; or wherein the plastisol has a viscosity ranging from 40,000 to 90,000 cP.
B11. The plastisol according to any of embodiments B7-B10, wherein the second plasticizer comprises phthalates; terephthalates; isophthalates; trimellitates; adipates; cyclohexanedicarboxylates; benzoates; phosphates; diesters of ethylene glycol, propylene glycol, their oligomers, and mixtures thereof; citrates; succinates; alkyl sulfonates; fatty acid esters and epoxidized fatty acid esters; triglycerides and epoxidized triglycerides, optionally substituted; dianhydrohexitol diesters; pentaerythritol-based tetraesters; furan-based esters; other esters; ketals; or polymeric plasticizers; or wherein the second plasticizer comprises dioctyl terephthalate, diisooctyl phthalate, di-2-ethylhexyl phthalate, di-2-ethylhexyl terephthalate, tri-2-ethylhexyl trimellitate, di-2-propylheptyl phthalate, diisononyl phthalate, diisodecyl phthalate, diisoundecyl phthalate, ditridecyl phthalate, trioctyl trimellitate, triisononyl trimellitate, 2,2,4-trimethyl-1,3-pentanediol diisobutyrate, isononyl benzoate, isodecyl benzoate, diisononyl 1,2-cyclohexanedicarboxylate, dioctyl adipate, di-2-ethylhexyl adipate, triethylene glycol di-2-ethylhexanoate, diethylene glycol dibenzoate, dipropylene glycol dibenzoate, or dibenzoates produced from mixtures of diethylene glycol and dipropylene glycol.
B12. The plastisol according to any of embodiments B7-B11, wherein the second plasticizer comprises dioctyl terephthalate, di-2-ethylhexyl terephthalate, dioctyl adipate, di-2-ethylhexyl adipate, or triethylene glycol di-2-ethylhexanoate; or wherein the second plasticizer comprises di-2-ethylhexyl terephthalate, diisononyl phthalate or diisononyl 1,2-cyclohexanedicarboxylate.
B13. The plastisol according to any of embodiments B7-B12, wherein the fillers comprises calcium carbonate, magnesium carbonate, silica, clay, mica, graphite, zinc oxide, or calcium oxide; or the fillers comprise calcium carbonate.
B14. The plastisol according to any of embodiments B7-B13, wherein the stabilizers comprises metal soaps, epoxidized oils and epoxidized fatty acid esters, or organotin compounds.
B15. The plastisol according to any of embodiments B7-B14, wherein the foaming agents comprise azodicarbonamide.
B16. The plastisol according to any of embodiments B7-B15, wherein the hollow materials comprise inorganic or organic glass beads, microbeads, or microspheres.
B17. The plastisol according to any of embodiments B7-B16, wherein the elastomeric materials comprise nitrile-butadiene rubber, natural rubber, isoprene rubber, butadiene rubber, styrene-butadiene rubber, butyl rubber, ethylene-propylene-diene monomer (EPDM) rubber, chloroprene rubber, styrenated block copolymers, ethylene-vinyl acetate copolymers, olefinic elastomers, olefinic copolymer elastomers, silicone elastomers, polysulfide elastomers, or polyurethane elastomers.
B18. The plastisol according to any of embodiments B7-B17, wherein the rheology control additives comprises petroleum distillates; mineral oil and mineral spirits; fatty acid esters; polyphenyl oligomers; or organic solvents.
B19. The plastisol according to any of embodiments B7-B18, wherein the adhesion promoters comprises polyamidoamines, blocked isocyanates and isocyanurates, silanes, or epoxy resins.
B20. The plastisol according to any of embodiments B1-B19, wherein the fused plastisol has a maximum Tan Delta (Tan δₘₐₓ) occurring between 20 °C and 50 °C and has Tan Delta at 30 °C (Tan δ_{30C}) ranging from 0.5 to 2.0, when measured on a sample nominally 0.6-0.7 mm thick, 3.2 mm wide, and 10-12 mm long using a Dynamic Mechanical Analyzer with a Tension Clamp at a strain of 0.1% and at a frequency of 1 Hz and a temperature ramp rate of 3 °C/min.
B21. The plastisol according to embodiment B20, wherein the Tan Delta at 30 °C (Tan δ_{30C}) ranges fom 0.5 to 1.8 or 0.5 to 1.6 or 0.5 to 1.4 or 0.6 to 2.0 or 0.6 to 1.8 or 0.6 to 1.6 or 0.6 to 1.4 or 0.7 to 2.0 or 0.7 to 1.8 or 0.7 to 1.6 or 0.7 to 1.4.
B22. The plastisol according to any of embodiments B20-B21, wherein the maximum Tan Delta (Tan δₘₐₓ) occurs between 20 °C and 40 °C or 30 °C and 50 °C.
C1. A method of improving vibration damping of a substrate comprising affixing a plastisol onto the substrate, wherein the plastisol comprises the plastisol according to any of embodiments B1-B22.
C2. The method according to embodiment C1, wherein the affixing comprises (a) applying the plastisol onto the substrate; (b) fusing the plastisol to produce a plastisol-covered substrate; and (c) cooling the plastisol-covered substrate to ambient temperatures.
C3. The method according to embodiment C2, wherein the applying the plastisol onto the substrate comprises coating the substrate with the plastisol.
C4. The method according to embodiment C3, wherein the coating comprises spray coating and/or extrusion coating.
C5. The method according to embodiments C1, wherein the affixing comprises (a) fusing the plastisol into a sheet; and (b) adhering the sheet to the substrate.
C6. The method according to any of embodiments C2-C5, wherein the fusing occurs at a temperature ranging from 100 °C to 220 °C for a time period ranging from 1 min to 2 hours; or at a temperature ranging from 140 °C to 180 °C for a time period ranging from 15 min. to 40 min.
C7. The method according to any of embodiments C1-C6, wherein the substrate is part of a wheeled vehicle.
C8. The method according to embodiment C7, wherein the substrate is on the underbody of the wheeled vehicle.

### Examples

The following plasticizers are commercially available and were used without further processing; Benzoflex™ 354 Plasticizer (Example 1a), Eastman 168™ Non-Phthalate Plasticizer (Comparative Example 1), Benzoflex™ 9-88 Plasticizer (Comparative Example 2) and Benzoflex™ 131 Plasticizer (Comparative Example 3) (Eastman Chemical Company, Kingsport, Tennessee), and Santicizer™ 278 Plasticizer (Comparative Example 4, Ferro Corporation, Mayfield, Ohio). All other ingredients used in the plastisols hereafter described are commercially available and were used without further processing.

The following plasticizer examples are used in the tables below.
Example 1a = 2,2,4-trimethyl-1,3-pentanediol dibenzoate (Benzoflex™ 354 Plasticizer)
Example 1b = 2,2,4-trimethyl-1,3-pentanediol ditoluate
Example 1c = 2,2,4-trimethyl-1,3-pentanediol benzoate/toluate
Example 1d = 2,4-dimethyl-2-ethyl-1,3-hexanediol dibenzoate
Example 1e = 2,4-dimethyl-2-ethyl-1,3-hexanediol ditoluate
Example 1f = 2,4-dimethyl-2-ethyl-1,3-hexanediol benzoate/toluate
Example 1g = 2-methyl-2-propyl-1,3-propanediol dibenzoate
Example 1h = 2-methyl-2-propyl-1,3-propanediol ditoluate
Example 1i = 2-methyl-2-propyl-1,3-propanediol benzoate/toluate
Example 1j = 2-butyl-2-ethyl-1,3-propanediol dibenzoate

### Examples 1(b) - 1(j):

To a 1 liter round bottomed flask flushed with nitrogen was charged 450 grams (3.0 moles) methyl toluate, 73.1 grams (0.5 moles) 2,2,4-trimethyl-1,3-pentanediol, and 4.81 grams (0.069 moles) potassium methoxide. The reaction mixture was heated to 220°C with continuous removal of methanol for three hours. At the end of the reaction, the mixture was cooled and the catalyst was neutralized with 4.12 grams (0.069 moles) acetic acid. Excess methyl toluate was removed under vacuum to yield 2,2,4-trimethyl-1,3-pentanediol ditoluate (Example 1b). Similarly, a mixture of methyl benzoate and methyl toluate was reacted with 2,2,4-trimethyl-1,3-pentanediol and potassium methoxide to yield 2,2,4-trimethyl-1,3-pentanediol benzoate/toluate (Example 1c).

To a 1 liter round bottomed flask flushed with nitrogen was charged 462 grams (3.78 moles) benzoic acid, 300 grams (1.72 moles) 2,4-dimethyl-2-ethyl-1,3-hexanediol, 4.89 grams (0.017 moles) titanium tetraisopropoxide, and 86 milliliters xylene. The reaction mixture was heated to 185°C with continuous removal of water for seven hours. After the mixture was cooled to 80°C, 200 milliliters 10% sodium hydroxide solution was added and the resulting mixture was stirred at 80°C for 30 minutes. The crude material was cooled and filtered through a pad of celite. The filtrate was washed successively with 10% sodium hydroxide solution, water, and brine solution. After drying over sodium sulfate and filtering, the crude product was purified through short-path distillation to yield 2,4-dimethyl-2-ethyl-1,3-hexanediol dibenzoate (Example 1d). Similarly, toluic acid was reacted with 2,4-dimethyl-2-ethyl-1,3-hexanediol and titanium tetraisopropoxide to yield 2,4-dimethyl-2-ethyl-1,3-hexanediol ditoluate (Example 1e), and a mixture of benzoic and toluic acids was reacted with 2,4-dimethyl-2-ethyl-1,3-hexanediol and titanium tetraisopropoxide to yield 2,4-dimethyl-2-ethyl-1,3-hexanediol benzoate/toluate (Example 1f).

To a 1 liter round bottomed flask flushed with nitrogen was charged 617 grams (4.54 moles) methyl benzoate, 150 grams (1.13 moles) 2-methyl-2-propyl-1,3-propanediol, and 7.96 grams (0.11 moles) potassium methoxide. The reaction mixture was heated to 220°C with continuous removal of methanol for five hours. At the end of the reaction, the mixture was cooled and the catalyst was neutralized with 6.78 grams (0.11 moles) acetic acid. Excess methyl benzoate was removed under vacuum to yield 2-methyl-2-propyl-1,3-propanediol dibenzoate (Example 1g). Similarly, methyl toluate was reacted with 2-methyl-2-propyl-1,3-propanediol and potassium methoxide to yield 2-methyl-2-propyl-1,3-propanediol ditoluate (Example 1h), and a mixture of methyl benzoate and methyl toluate was reacted with 2-methyl-2-propyl-1,3-propanediol and potassium methoxide to yield 2-methyl-2-propyl-1,3-propanediol benzoate/toluate (Example 1i). Similarly, methyl benzoate was reacted with 2-butyl-2-ethyl-1,3-propanediol and potassium methoxide to yield 2-butyl-2-ethyl-1,3-propanediol dibenzoate (Example 1j).

### Preparation of PVC Formulations and Samples for DMTA Evaluation

### Example 2

A FlackTek SpeedMixer™ model 150FV was used to prepare PVC plastisols. To a mixing cup was added 7 grams Geon™ 121A PVC paste resin, 3 grams Geon™ 217 PVC blending resin, 4 grams UltraPflex™ precipitated calcium carbonate, 8 grams Hubercarb™ Q325 calcium carbonate, 0.4 grams calcium oxide, 0.2 grams zinc oxide, 1.0 grams Varsol™ 18 Non-dearomatized Fluid, and 15 grams of Benzoflex™ 354 (Example 1(a)) Plasticizer. The contents were shaken in the mixer for 45 seconds and the side of the container was scraped. This process was repeated twice to ensure complete dispersion. The resulting plastisol was then deaerated in a desiccator to which vacuum was applied for 20 minutes.

Samples for DMTA analysis were prepared by drawdowns of the deaerated plastisols onto release paper at a 25 mil thickness, then fused at 350°F for 25 minutes. Dynamic Mechanical Thermal Analysis (DMTA) measurements were performed on these samples using a tension clamp on a DMA Q800 from TA Instruments. Samples were cut using a 1/8 inch precision cutter, and sample width and thickness were recorded into the software. After loading the sample into the tension clamps, the software measured and recorded sample length. A 0.1 % strain was placed on the sample at a 1 Hz frequency. The sample was then cooled with liquid nitrogen to -100°C. Once the temperature equilibrated, the sample was heated at a 3°C per minute rate until a maximum of 100°C was reached. Storage modulus, loss modulus, and tan δ results were recorded. Tan δ results at temperatures from -30° C to 50° C, in 10° C increments, are given in Table 1b.

### Examples 3 - 12 and Comparative Examples C5 - C9

Example 4 was repeated, using the type and amount of plasticizer and the amount of Varsol rheology control additive as indicated in Table 1a. The correspondent tan δ results are given in Table 1b.

**Table 1a - Plastisols: 7 parts Geon™ 121A, 3 parts Geon™ 217, 4 parts UltraPflex™, 8 parts Q325, 0.4 parts calcium oxide, 0.2 parts zinc oxide, plasticizers and optionally Varsol™ 18 rheology control additive as noted**

| Example | Plasticizer Example | Parts Plasticizer | Parts Varsol |
|---|---|---|---|
| 2(a) | 1a | 15 | 1 |
| 3(a) | 1b | 15 | 1 |
| 4(a) | 1c | 15 | 1 |
| 5(a) | 1d | 15 | 1 |
| 6 | 1e | 15 | 1 |
| 7 | 1f | 15 | 0 |
| 8(a) | 1g | 15 | 1 |
| 9 | 1h | 15 | 1 |
| 10 | 1i | 15 | 1 |
| 11 | 1j | 15 | 1 |
| 12 | Blend of 1a/Comparative Example 3 | 7.5/7.5 | 0 |
| C5 | Comparative Example 1 | 15 | 0 |
| C6 | Comparative Example 2 | 15 | 0 |
| C7 | Comparative Example 3 | 12 | 0 |
| C8(a) | Comparative Example 3 | 10 | 0 |
| C9(a) | Comparative Example 4 | 15 | 0 |

**Table 1b - Tan Delta**

| | | Tan Delta (Tan δ) at given temperature (°C) | | | | | | | | |
|---|---|---|---|---|---|---|---|---|---|---|
| Example | Plasticizer Example | -30 | -20 | -10 | 0 | 10 | 20 | 30 | 40 | 50 |
| 2(a) | 1a | 0.03 | 0.07 | 0.19 | 0.43 | 0.71 | 0.99 | 0.73 | 0.35 | 0.19 |
| 3(a) | 1b | 0.02 | 0.03 | 0.05 | 0.12 | 0.37 | 0.84 | 1.47 | 0.81 | 0.34 |
| 4(a) | 1c | 0.03 | 0.04 | 0.09 | 0.27 | 0.65 | 1.25 | 1.08 | 0.48 | 0.22 |
| 5(a) | 1d | 0.03 | 0.06 | 0.12 | 0.35 | 0.73 | 1.24 | 0.92 | 0.41 | 0.20 |
| 6 | 1e | 0.05 | 0.11 | 0.22 | 0.30 | 0.32 | 0.32 | 0.31 | 0.29 | 0.27 |
| 7 | 1f | 0.03 | 0.06 | 0.12 | 0.30 | 0.55 | 0.79 | 0.95 | 0.57 | 0.26 |
| 8(a) | 1g | 0.04 | 0.07 | 0.19 | 0.53 | 1.05 | 1.10 | 0.50 | 0.23 | 0.14 |
| 9 | 1h | 0.04 | 0.05 | 0.08 | 0.23 | 0.68 | 1.50 | 0.96 | 0.39 | 0.20 |
| 10 | 1i | 0.02 | 0.04 | 0.10 | 0.34 | 0.81 | 1.35 | 0.78 | 0.32 | 0.17 |
| 11 | 1j | 0.02 | 0.05 | 0.14 | 0.43 | 0.91 | 1.12 | 0.53 | 0.24 | 0.14 |
| 12 | Blend of 1a/Comparative Example 3 | 0.15 | 0.24 | 0.33 | 0.41 | 0.48 | 0.54 | 0.49 | 0.31 | 0.18 |
| C5 | Comparative Example 1 | 0.59 | 0.82 | 0.56 | 0.33 | 0.21 | 0.14 | 0.11 | 0.09 | 0.08 |
| C6 | Comparative Example 2 | 0.07 | 0.21 | 0.44 | 0.70 | 0.85 | 0.53 | 0.28 | 0.17 | 0.13 |
| C7 | Comparative Example 3 | 0.17 | 0.19 | 0.22 | 0.25 | 0.29 | 0.32 | 0.32 | 0.29 | 0.26 |
| C8(a) | Comparative Example 3 | 0.18 | 0.20 | 0.22 | 0.24 | 0.27 | 0.29 | 0.29 | 0.26 | 0.24 |
| C9(a) | Comparative Example 4 | 0.09 | 0.12 | 0.25 | 0.58 | 1.02 | 0.89 | 0.46 | 0.25 | 0.16 |

For a limited number of the plastisols listed in Table 1a, the plastisol formulation was made a second time with the addition of 0.6 parts of Nourybond 272 (an adhesive promoter). These plastisols were subject to the Oberst bar method test.

The test was conducted as follows. Measurements were in accordance with the test method described in the SAE Recommended Practice J1637-07, *Laboratory Measurement of Composite Vibration Damping Properties of Materials on a Supporting Steel Bar.*
The nominal dimensions of the steel bars were: mounted free length 200 mm, thickness 0.8 mm, and width 12.7 mm. Visco-elastic (damping) materials were bonded to this bar. Any excess material bonded to the bar was removed and cleaned, prior to installing the bar on the test set-up. The test temperatures that were used for this study were 10°C, 25°C, and 40°C.

Tests were made on a fixture to measure various modes of vibration that were generally between 100 Hz and 1000 Hz for each test bar using a random noise signal. The resonant frequency and the half power bandwidth (frequency difference between 3 dB down points from the resonant peak) of each mode needed for composite loss factor computation were read directly from a Pulse Multi-Analyzer System Type 3560. When the 3 dB down points on both sides of the resonant frequency were not measurable, the "n" dB down point method was used wherever possible per SAE Standard J1637-07.

The Oberst bar composite loss factors are shown as interpolated values at 200 Hz, 400 Hz, and 800 Hz for each temperature. These values are based on linear interpolation of two sets of data points where the frequency and the loss factor information are provided in a logarithmic scale. Should it not have been possible to interpolate data, then it was extrapolated. Results are shown in Table 2.

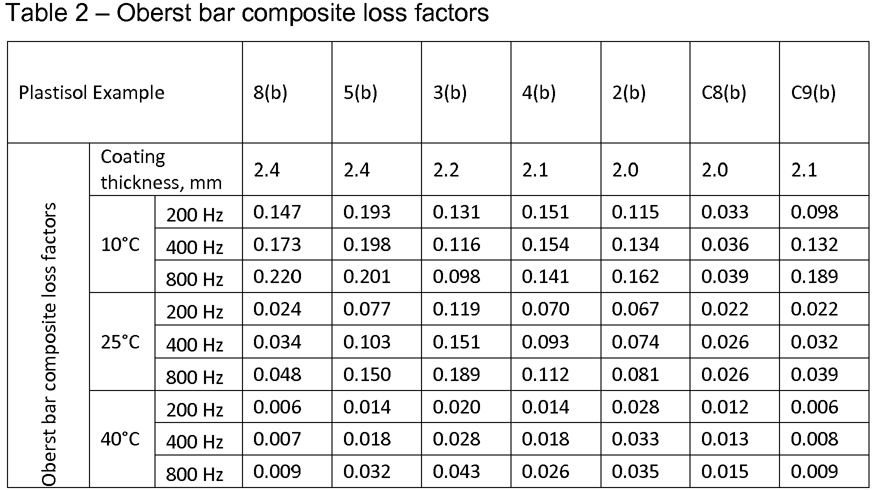

### Example 13

A FlackTek SpeedMixer™ model 150FV was used to prepare PVC plastisols. To a mixing cup was added 10 grams Geon™ 121A PVC paste resin, 4 grams Geon™ 217 PVC blending resin, 6 grams UltraPflex™ precipitated calcium carbonate, 0.4 grams calcium oxide, 0.2 grams zinc oxide, 1.0 grams Varsol 18™ Non-dearomatized Fluid, and 10 grams of Benzoflex™ 354 (Example 1(a)) Plasticizer. The contents were shaken in the mixer for 45 seconds and the side of the container was scraped. This process was repeated twice to ensure complete dispersion. The resulting plastisol was then deaerated in a desiccator to which vacuum was applied for 20 minutes Tan δ results at temperatures from -30° C to 50° C, in 10° C increments, are given in Table 3b.

### Examples 14 - 32 and Comparative Examples C10 - C15

Example 13 was repeated, using the type and amount of plasticizer and the amount of Varsol rheology control additive as indicated in Table 3a. The correspondent tan δ results are given in Table 3b.

**Table 3a - Plastisols: 10 parts Geon™ 121A, 4 parts Geon™ 217, 6 parts UltraPflex™, 0.4 parts calcium oxide, 0.2 parts zinc oxide, plasticizers and optionally Varsol™ 18 rheology control additive as noted**

| Example | Plasticizer Example | Parts Plasticizer | Parts Varsol |
|---|---|---|---|
| 13 | 1a | 16 | 1 |
| 14(a) | 1a | 14 | 1 |
| 15(a) | 1a | 10 | 2.5 |
| 16 | 1b | 16 | 1 |
| 17 | 1c | 16 | 1 |
| 18(a) | 1c | 14 | 2 |
| 19 | 1d | 13 | 2 |
| 20(a) | 1d | 14 | 2 |
| 21 | 1e | 13 | 1 |
| 22(a) | 1e | 14 | 1 |
| 23 | 1f | 13 | 2 |
| 24(a) | 1f | 14 | 2 |
| 25(a) | 1g | 10 | 2.5 |
| 26(a) | 1h | 10 | 2.5 |
| 27(a) | 1i | 10 | 2.5 |
| 28(a) | 1j | 10 | 2 |
| 29 | Blend of 1a/Comparative Example 1 | 6/6 | 1 |
| 30(a) | Blend of 1a/Comparative Example 1 | 5/5 | 1 |
| 31 | Blend of 1a/Comparative Example 3 | 8/8 | 0 |
| 32(a) | Blend of 1a/Comparative Example 3 | 7/7 | 0 |
| C10 | Comparative Example 1 | 10 | 1 |
| C11 | Comparative Example 1 | 8 | 2 |
| C12(a) | Comparative Example 1 | 6 | 3 |
| C13 | Comparative Example 2 | 10 | 2 |
| C14(a) | Comparative Example 4 | 10 | 2 |
| C15(a) | Comparative Example 4 | 8 | 2.5 |

**Table 3b - Tan Delta**

| | | Tan Delta (Tan δ) at given temperature (°C) | | | | | | | | |
|---|---|---|---|---|---|---|---|---|---|---|
| Example | Plasticizer Example | -30 | -20 | -10 | 0 | 10 | 20 | 30 | 40 | 50 |
| 13 | 1a | 0.04 | 0.05 | 0.12 | 0.26 | 0.45 | 0.60 | 0.75 | 0.65 | 0.32 |
| 14(a) | 1a | 0.05 | 0.06 | 0.09 | 0.17 | 0.32 | 0.44 | 0.50 | 0.56 | 0.46 |
| 15(a) | 1a | 0.05 | 0.07 | 0.10 | 0.16 | 0.27 | 0.39 | 0.47 | 0.55 | 0.51 |
| 16 | 1b | 0.03 | 0.03 | 0.04 | 0.08 | 0.18 | 0.47 | 1.05 | 1.28 | 0.55 |
| 17 | 1c | 0.04 | 0.04 | 0.07 | 0.15 | 0.38 | 0.78 | 1.18 | 0.68 | 0.29 |
| 18(a) | 1c | 0.04 | 0.05 | 0.08 | 0.17 | 0.37 | 0.66 | 0.99 | 0.72 | 0.30 |
| 19 | 1d | 0.04 | 0.05 | 0.07 | 0.13 | 0.27 | 0.49 | 0.80 | 0.89 | 0.46 |
| 20(a) | 1d | 0.04 | 0.06 | 0.10 | 0.18 | 0.35 | 0.60 | 0.88 | 0.71 | 0.32 |
| 21 | 1e | 0.04 | 0.06 | 0.10 | 0.16 | 0.21 | 0.24 | 0.24 | 0.22 | 0.21 |
| 22(a) | 1e | 0.06 | 0.10 | 0.16 | 0.23 | 0.27 | 0.29 | 0.29 | 0.26 | 0.22 |
| 23 | 1f | 0.03 | 0.04 | 0.06 | 0.11 | 0.21 | 0.36 | 0.52 | 0.70 | 0.67 |
| 24(a) | 1f | 0.04 | 0.06 | 0.10 | 0.19 | 0.33 | 0.46 | 0.54 | 0.63 | 0.49 |
| 25(a) | 1g | 0.04 | 0.05 | 0.07 | 0.13 | 0.27 | 0.48 | 0.76 | 0.77 | 0.36 |
| 25(a) | 1h | 0.04 | 0.05 | 0.05 | 0.07 | 0.12 | 0.22 | 0.49 | 0.98 | 0.82 |
| 27(a) | 1i | 0.04 | 0.05 | 0.06 | 0.10 | 0.19 | 0.39 | 0.73 | 0.91 | 0.44 |
| 28(a) | 1j | 0.04 | 0.05 | 0.08 | 0.14 | 0.26 | 0.49 | 0.77 | 0.72 | 0.35 |
| 29 | Blend of 1a/Comparative Example 1 | 0.13 | 0.19 | 0.28 | 0.39 | 0.50 | 0.61 | 0.53 | 0.32 | 0.19 |
| 30(a) | Blend of 1a/Comparative Example 1 | 0.07 | 0.12 | 0.19 | 0.27 | 0.37 | 0.49 | 0.55 | 0.42 | 0.25 |
| 31 | Blend of 1a/Comparative Example 3 | 0.10 | 0.16 | 0.25 | 0.33 | 0.39 | 0.47 | 0.51 | 0.38 | 0.20 |
| 32(a) | Blend of 1a/Comparative Example 3 | 0.09 | 0.14 | 0.21 | 0.29 | 0.36 | 0.40 | 0.41 | 0.37 | 0.28 |
| C10 | Comparative Example 1 | 0.16 | 0.21 | 0.26 | 0.31 | 0.37 | 0.40 | 0.36 | 0.27 | 0.19 |
| C11 | Comparative Example 1 | 0.12 | 0.15 | 0.18 | 0.22 | 0.26 | 0.32 | 0.37 | 0.36 | 0.29 |
| C12(a) | Comparative Example 1 | 0.07 | 0.09 | 0.11 | 0.14 | 0.18 | 0.23 | 0.29 | 0.36 | 0.39 |
| C13 | Comparative Example 2 | 0.03 | 0.05 | 0.10 | 0.19 | 0.31 | 0.45 | 0.61 | 0.55 | 0.29 |
| C14(a) | Comparative Example 4 | 0.04 | 0.05 | 0.08 | 0.14 | 0.25 | 0.45 | 0.70 | 0.74 | 0.41 |
| C15(a) | Comparative Example 4 | 0.04 | 0.05 | 0.06 | 0.09 | 0.15 | 0.27 | 0.49 | 0.74 | 0.59 |

For a limited number of the plastisols listed in Table 3a, the plastisol formulation was made a second time with the addition of 0.6 parts of Nourybond 272 (an adhesive promoter). These plastisols were subject to the Oberst bar method test as described previously. Results are shown in Tables 4-5.

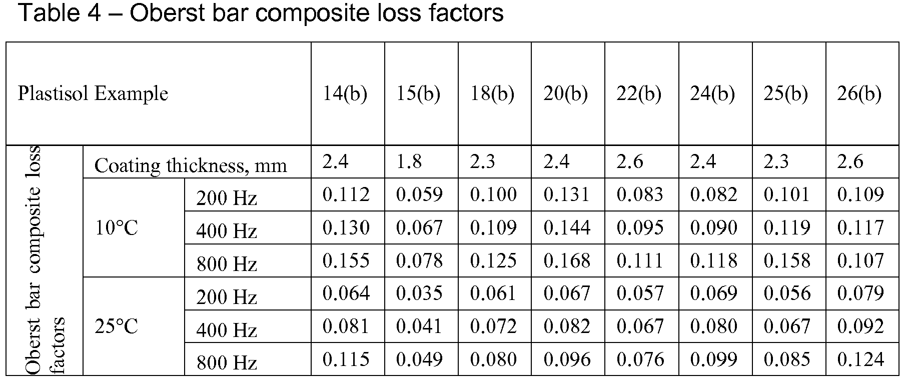

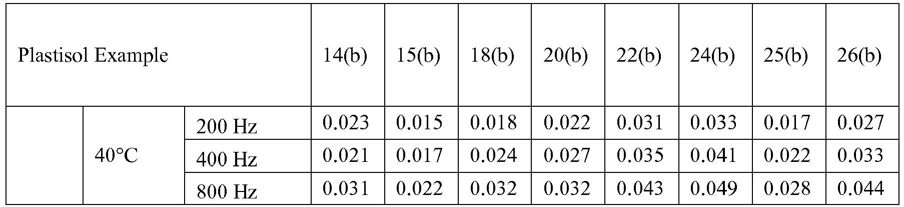

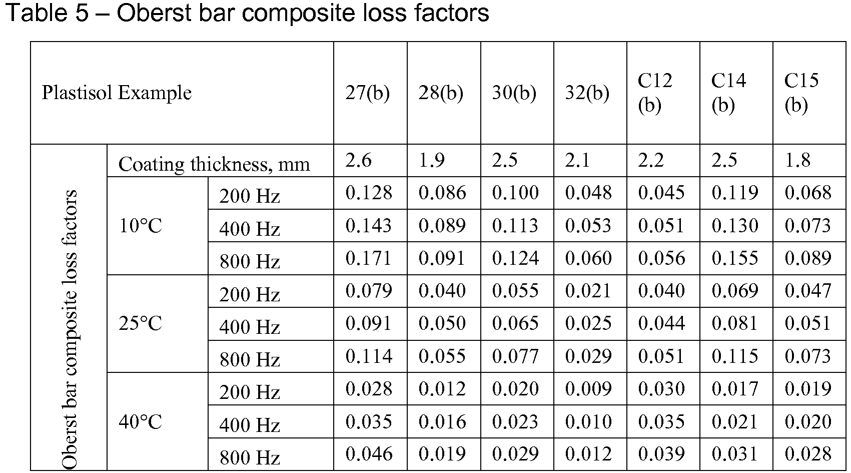

### Example 33

A FlackTek SpeedMixer™ model 150FV was used to prepare PVC plastisols. To a mixing cup was added 10 grams Geon™ 121A PVC paste resin, 4 grams Geon™ 217 PVC blending resin, 6 grams UltraPflex™ precipitated calcium carbonate, 0.4 grams calcium oxide, 0.2 grams zinc oxide, 1.0 grams Varsol 18™ Non-dearomatized Fluid, 0.1 grams azodicarbonamide, and 16 grams of Benzoflex™ 354 (Example 1(a)) Plasticizer. The contents were shaken in the mixer for 45 seconds and the side of the container was scraped. This process was repeated twice to ensure complete dispersion. The resulting plastisols were then deaerated in a desiccator to which vacuum was applied for 20 minutes. Tan δ results at temperatures from -30° C to 50° C, in 10° C increments, are given in Table 6b.

### Examples 34 - 35

Example 33 was repeated, using the type and amount of plasticizer, the amount of rheology control additive, and the type and amount of foaming agent as indicated in Table 6a. The correspondent tan δ results are given in Table 6b.

**Table 6a - Examples with foaming agent**

| Plastisols: 10 parts Geon™ 121A, 4 parts Geon™ 217, 6 parts UltraPflex™, 0.4 parts calcium oxide, 0.2 parts zinc oxide, plasticizers, Varsol™ 18 rheology control additive, and foaming agent as noted | | | | |
|---|---|---|---|---|
| Example | Plasticizer Example | Parts Plasticizer | Parts Varsol | Parts foaming agent |
| 33 | 1a | 16 | 1 | 0.1 (azodicarbonamide) |
| 34 | 1a | 16 | 1.5 | 0.5 (expandable poly beads) |
| 35 | 1a | 16 | 1.5 | 1.0 (expandable poly beads) |

**Table 6b - Tan Delta**

| | | Tan Delta (Tan δ) at given temperature (°C) | | | | | | | | |
|---|---|---|---|---|---|---|---|---|---|---|
| Example | Plasticizer/Foaming Agent | -30 | -20 | -10 | 0 | 10 | 20 | 30 | 40 | 50 |
| 33 | 1a/0.1 parts azodicarbonamide | 0.04 | 0.05 | 0.10 | 0.21 | 0.42 | 0.65 | 0.85 | 0.63 | 0.29 |
| 34 | 1a/0.5 parts poly beads | 0.08 | 0.12 | 0.21 | 0.36 | 0.47 | 0.51 | 0.42 | 0.27 | 0.16 |
| 35 | 1a/1.0 parts poly beads | 0.18 | 0.22 | 0.29 | 0.39 | 0.44 | 0.43 | 0.31 | 0.19 | 0.13 |

Other embodiments will be apparent to those skilled in the art from consideration of the specification and practice of the embodiments disclosed herein.

## Claims

1. A method of improving vibration damping of a substrate comprising affixing a plastisol onto said substrate, wherein said plastisol comprises a polymeric component and a plasticizer, wherein said plasticizer comprises the esterification product formed by the reaction of formula (I) 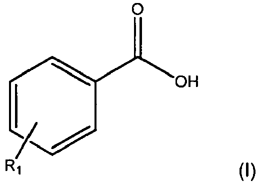 and formula (II) 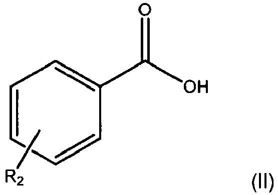 or the corresponding esters, anhydrides, and/or acid chlorides of formula (I) and/or formula (II), with formula (III) 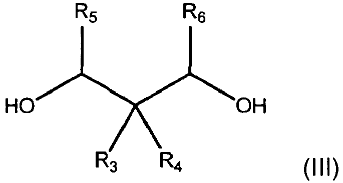 wherein R¹ and R² are independently selected from the group consisting of hydrogen and linear or branched alkyl groups containing 1 to 9 carbon atoms; R³ and R⁴ are independently selected from the group consisting of hydrogen and linear or branched alkyl groups having 1 to 8 carbon atoms whereby the sum of the number of carbon atoms of R³ and R⁴ is from 0 to 10; and R⁵ and R⁶ are independently selected from the group consisting of hydrogen and linear or branched alkyl groups having 1 to 8 carbon atoms whereby the sum of the number of carbon atoms of R⁵ and R⁶ is from 0 to 10.

2. The method according to claim 1, with the proviso that R¹ and R² are not each hydrogen.

3. The method according to claim 1 or 2, wherein said plasticizer comprises 2,2,4-trimethyl-1,3-pentanediol ditoluate, 2,2,4-trimethyl-1,3-pentanediol benzoate/toluate, 2,2,4-trimethyl-1,3-pentanediol dibenzoate, 2,4-dimethyl-2-ethyl-1,3-hexanediol ditoluate, 2,4-dimethyl-2-ethyl-1,3-hexanediol benzoate/toluate, 2,4-dimethyl-2-ethyl-1,3-hexanediol dibenzoate, 2-methyl-2-propyl-1,3-propanediol ditoluate, 2-methyl-2-propyl-1,3-propanediol benzoate/toluate, 2-methyl-2-propyl-1,3-propanediol dibenzoate, 2-butyl-2-ethyl-1,3-propanediol dibenzoate, 2-butyl-2-ethyl-1,3-propanediol ditoluate, 2-butyl-2-ethyl-1,3-propanediol benzoate/toluate or 2-butyl-2-ethyl-1,3-propanediol dibenzoate.

4. The method according to any of claims 1-3, wherein said plasticizer comprises said esterification product formula (IV) 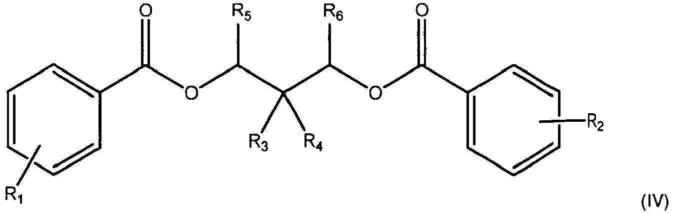 and wherein said esterification product comprises at least 10 weight percent of a mixed ester, based on the total weight of said esterification product.

5. The method according to any of claims 1-4, comprising 10 weight percent to 70 weight percent of said plasticizer, 10 weight percent to 70 weight percent of said polymeric component, and 10 weight percent to 80 weight percent of other components, each based on the total weight of said plastisol, wherein said other components comprise a second plasticizer, fillers, pigments, stabilizers, foaming agents, hollow materials, elastomeric materials, rheology control additives, or adhesion promoters, and wherein said polymeric component comprises polyvinyl chloride, polyvinyl acetate, acrylic polymers, and/or vinyl chloride-containing copolymers.

6. The method according to claim 5, wherein said second plasticizer comprises phthalates; terephthalates; isophthalates; trimellitates; adipates; cyclohexanedicarboxylates; benzoates; phosphates; diesters of ethylene glycol, propylene glycol, their oligomers, and mixtures thereof; citrates; succinates; alkyl sulfonates; fatty acid esters and epoxidized fatty acid esters; triglycerides and epoxidized triglycerides, optionally substituted; dianhydrohexitol diesters; pentaerythritol-based tetraesters; furan-based esters; other esters; ketals; or polymeric plasticizers.

7. The method according to any of claims 5-6, wherein said fillers comprise calcium carbonate, magnesium carbonate, silica, clay, mica, graphite, zinc oxide, or calcium oxide; or said fillers comprise calcium carbonate.

8. The method according to any of claims 5-7, wherein said rheology control additives comprise petroleum distillates; mineral oil and mineral spirits; fatty acid esters; polyphenyl oligomers; or organic solvents.

9. The method according to any of claims 1-8, wherein said fused plastisol has a maximum Tan Delta (Tan δₘₐₓ) occurring between 20 °C and 50 °C and has Tan Delta at 30 °C (Tanδ_{30C}) ranging from 0.5 to 2.0, when measured on a sample nominally 0.6-0.7 mm thick, 3.2 mm wide, and 10-12 mm long using a Dynamic Mechanical Analyzer with a Tension Clamp at a strain of 0.1 % and at a frequency of 1 Hz and a temperature ramp rate of 3 °C/min.

10. The method according to claim 9, wherein said maximum Tan Delta (Tan δₘₐₓ) occurs between 20 °C and 40 °C.

11. The method according to any of claims 1-10, wherein said affixing comprises
a. applying said plastisol onto said substrate, wherein said applying said plastisol onto said substrate comprises coating said substrate with said plastisol, and wherein said coating comprises spray coating and/or extrusion coating;
b. fusing said plastisol to produce a plastisol-covered substrate; and
c. cooling said plastisol-covered substrate to ambient temperatures.

12. The method according to any of claims 1-11, wherein said affixing comprises
a. fusing said plastisol into a sheet; and
b. adhering said sheet to said substrate.

13. The method according to any of claims 1-12, wherein said substrate is part of a wheeled vehicle.

## Patentansprüche

1. Verfahren zur Verbesserung der Schwingungsdämpfung eines Substrats, umfassend das Anbringen eines Plastisols auf dem Substrat, wobei das Plastisol eine polymere Komponente und einen Weichmacher umfasst, wobei der Weichmacher das durch die Reaktion der Formel (I) 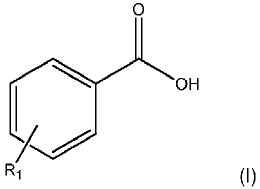 und Formel (II) 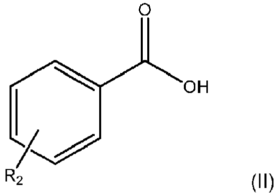 gebildete Veresterungsprodukt umfasst,
oder die entsprechenden Ester, Anhydride und / oder Säurechloride der Formel (I) und / oder Formel (II) mit der Formel (III) 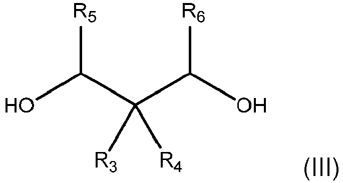 worin R¹ und R² unabhängig ausgewählt sind aus der Gruppe bestehend aus Wasserstoff und linearen oder verzweigten Alkylgruppen mit 1 bis 9 Kohlenstoffatomen; R³ und R⁴ unabhängig ausgewählt sind aus der Gruppe bestehend aus Wasserstoff und linearen oder verzweigten Alkylgruppen mit 1 bis 8 Kohlenstoffatomen, wobei die Summe der Anzahl der Kohlenstoffatome von R³ und R⁴ 0 bis 10 ist; und R⁵ und R⁶ unabhängig ausgewählt sind aus der Gruppe bestehend aus Wasserstoff und linearen oder verzweigten Alkylgruppen mit 1 bis 8 Kohlenstoffatomen, wobei die Summe der Anzahl der Kohlenstoffatome von R⁵ und R⁶ 0 bis 10 beträgt.

2. Verfahren nach Anspruch 1, mit der Maßgabe, dass R¹ und R² nicht jeweils Wasserstoff sind.

3. Verfahren nach Anspruch 1 oder 2, wobei der Weichmacher umfasst: 2,2,4-Trimethyl-1,3-pentandiol-ditoluat, 2,2,4-Trimethyl-1,3-pentandiolbenzoat / -toluat, 2,2,4-Trimethyl-1,3-pentandioldibenzoat, 2,4-Dimethyl-2-ethyl-1,3-hexandiolditoluat, 2,4-Dimethyl-2-ethyl-1,3-hexandiolbenzoat / -toluat, 2,4-Dimethyl-2-ethyl-1,3-hexandioldibenzoat, 2-Methyl-2-propyl-1,3-propandiolditoluat, 2-Methyl-2-propyl-1,3-propandiolbenzoat / -toluat, 2-Methyl-2-propyl-1,3-propandioldibenzoat, 2-Butyl-2-ethyl-1,3-propandioldibenzoat, 2-Butyl-2-ethyl-1,3-propandiolditoluat, 2-Butyl-2-ethyl-1,3-propandiolbenzoat / -toluat oder 2-Butyl-2-ethyl-1,3-propandioldibenzoat.

4. Verfahren nach einem der Ansprüche 1-3, wobei der Weichmacher das Veresterungsprodukt der Formel (IV) umfasst 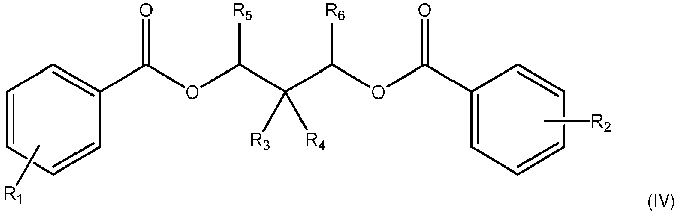 und wobei das Veresterungsprodukt mindestens 10 Gewichtsprozent eines gemischten Esters umfasst, bezogen auf das Gesamtgewicht des Veresterungsprodukts.

5. Verfahren nach einem der Ansprüche 1 bis 4, umfassend 10 Gew.-% bis 70 Gew.-% des Weichmachers, 10 Gew.-% bis 70 Gew.-% der polymeren Komponente und 10 Gew.-% bis 80 Gew.-% der anderen Komponenten, jeweils bezogen auf das Gesamtgewicht des Plastisols, wobei die anderen Komponenten einen zweiten Weichmacher, Füllstoffe, Pigmente, Stabilisatoren, Schaumbildner, hohle Materialien, elastomere Materialien, Rheologiesteuerungsadditive oder Haftvermittler umfassen, und wobei die polymere Komponente umfasst: Polyvinylchlorid, Polyvinylacetat, Acrylpolymere und / oder Vinylchlorid enthaltende Copolymere.

6. Verfahren nach Anspruch 5, wobei der zweite Weichmacher umfasst: Phthalate; Terephthalate; Isophthalate; Trimellitate; Adipate; Cyclohexandicarboxylate; Benzoate; Phosphate; Diester von Ethylenglykol, Propylenglykol, ihren Oligomeren und Gemischen davon; Citrate; Succinate; Alkylsulfonate; Fettsäureester und epoxidierte Fettsäureester; Triglyceride und epoxidierte Triglyceride, gegebenenfalls substituiert; Dianhydrohexitoldiester; Tetraester auf Pentaerythrit-Basis; Furanbasierte Ester; andere Ester; Ketale; oder polymere Weichmacher.

7. Verfahren nach einem der Ansprüche 5 bis 6, wobei die Füllstoffe umfassen: Calciumcarbonat, Magnesiumcarbonat, Siliciumdioxid, Ton, Glimmer, Graphit, Zinkoxid oder Calciumoxid; oder wobei die Füllstoffe Calciumcarbonat umfassen.

8. Verfahren nach einem der Ansprüche 5 bis 7, wobei die rheologiesteuernden Additive umfassen: Erdöldestillate; Mineralöl und Testbenzin; Fettsäureester; Polyphenyloligomere; oder organische Lösungsmittel.

9. Verfahren nach einem der Ansprüche 1 bis 8, wobei das geschmolzene Plastisol ein maximales Tan Delta (Tan δₘₐₓ) aufweist, das zwischen 20 °C und 50 °C auftritt und ein Tan Delta bei 30 °C (Tanδ_{30C}) im Bereich von 0,5 bis 2,0 aufweist, gemessen an einer Probe von nominal 0,6-0,7 mm Dicke, 3,2 mm Breite und 10-12 mm Länge, unter Verwendung eines dynamischen mechanischen Analysators mit einer Spannungsklemme bei einer Belastung von 0,1% und einer Frequenz von 1 Hz und einer Temperaturrampenrate von 3 °C/min.

10. Verfahren nach Anspruch 9, wobei das maximale Tan Delta (Tan δₘₐₓ) zwischen 20 °C und 40 °C auftritt.

11. Verfahren nach einem der Ansprüche 1-10, wobei das Anbringen umfasst
a. Auftragen des Plastisols auf das Substrat, wobei das Auftragen des Plastisols auf das Substrat das Beschichten des Substrats mit dem Plastisol umfasst und wobei die Beschichtung Sprühbeschichten und / oder Extrusionsbeschichten umfasst;
b. Schmelzen des Plastisols, um ein mit Plastisol bedecktes Substrat zu erzeugen; und
c. Abkühlen des mit Plastisol bedeckten Substrats auf Umgebungstemperaturen.

12. Verfahren nach einem der Ansprüche 1-11, wobei das Anbringen umfasst
a. Schmelzen des Plastisols zu einer Folie; und
b. Ankleben der Folie an das Substrat.

13. Verfahren nach einem der Ansprüche 1-12, wobei das Substrat Teil eines Fahrzeugs mit Rädern ist.

## Revendications

1. Procédé d'amélioration de l'amortissement des vibrations d'un substrat comprenant l'apposition d'un plastisol sur ledit substrat, dans lequel ledit plastisol comprend un composant polymère et un plastifiant, où ledit plastifiant comprend le produit d'estérification formé par réaction de la formule (I) 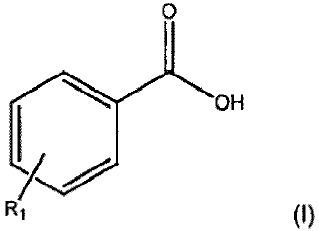 et de la formule (II) 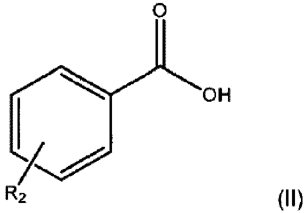 ou des esters, anhydrides, et/ou chlorures d'acides de formule (I) et/ou de formule (II) correspondants, avec la formule (III) 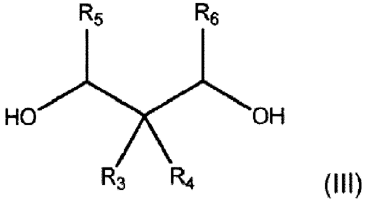 où R¹ et R² sont indépendamment choisis dans le groupe constitué par un atome d'hydrogène et les groupes alkyle linéaires ou ramifiés contenant de 1 à 9 atomes de carbone ; R³ et R⁴ sont indépendamment choisis dans le groupe constitué par un atome d'hydrogène et les groupes alkyle linéaires ou ramifiés contenant de 1 à 8 atomes de carbone de façon que la somme du nombre d'atomes de carbone de R³ et R⁴ est comprise entre 0 et 10 ; et R⁵ et R⁶ sont indépendamment choisis dans le groupe constitué par un atome d'hydrogène et les groupes alkyle linéaires ou ramifiés contenant de 1 à 8 atomes de carbone de façon que la somme du nombre d'atomes de carbone de R⁵ et R⁶ est comprise entre 0 et 10.

2. Procédé selon la revendication 1, à condition que R¹ et R² ne sont pas chacun un atome d'hydrogène.

3. Procédé selon la revendication 1 ou 2, dans lequel ledit plastifiant comprend le ditoluate de 2,2,4-triméthyl-1,3-pentanediol, le benzoate/toluate de 2,2,4-triméthyl-1,3-pentanediol, le dibenzoate de 2,2,4-triméthyl-1,3-pentanediol, le ditoluate de 2,4-diméthyl-2-éthyl-1,3-hexanediol, le benzoate/toluate de 2,4-diméthyl-2-éthyl-1,3-hexanediol, le dibenzoate de 2,4-diméthyl-2-éthyl-1,3-hexanediol, le ditoluate de 2-méthyl-2-propyl-1,3-propanediol, le benzoate/ toluate de 2-méthyl-2-propyl-1,3-propanediol, le dibenzoate de 2-méthyl-2-propyl-1,3-propanediol, le dibenzoate de 2-butyl-2-éthyl-1,3-propanediol, le ditoluate de 2-butyl-2-éthyl-1,3-propanediol, ou le benzoate/toluate de 2-butyl-2-éthyl-1,3-propanediol ou le dibenzoate de 2-butyl-2-éthyl-1,3-propanediol.

4. Procédé selon l'une quelconque des revendications 1 à 3, dans lequel ledit plastifiant comprend ledit produit d'estérification de formule (IV) 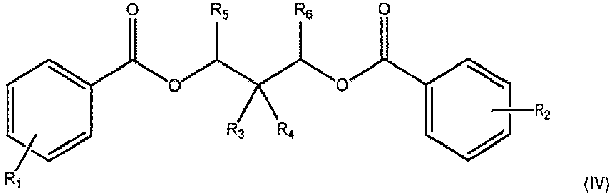 et dans lequel ledit produit d'estérification comprend au moins 10 % en poids d'un ester mixte, sur la base du poids total dudit produit d'estérification.

5. Procédé selon l'une quelconque des revendications 1 à 4, comprenant de 10 à 70 % en poids dudit plastifiant, de 10 à 70 % en poids dudit composant polymère, et de 10 à 80 % en poids d'autres composants, chacun sur la base du poids total dudit plastisol, où lesdits autres composants comprennent un second plastifiant, des charges, des pigments, des stabilisants, des agents d'expansion, des matériaux creux, des matières élastomères, des additifs de régulation de rhéologie, ou des promoteurs d'adhérence, et où ledit composant polymère comprend le polychlorure de vinyle, le polyacétate de vinyle, les polymères acryliques, et/ou les copolymères contenant du chlorure de vinyle.

6. Procédé selon la revendication 5, dans lequel ledit second plastifiant comprend les phtalates ; les téréphtalates ; les isophtalates ; les trimellitates ; les adipates ; les cyclohexanedicarboxylates ; les benzoates ; les phosphates ; les diesters d'éthylène glycol, propylène glycol, leurs oligomères, et leurs mélanges ; les citrates ; les succinates ; les sulfonates d'alkyle ; les esters d'acides gras et esters d'acides gras époxydés ; les triglycérides et triglycérides époxydés, éventuellement substitués ; les diesters de dianhydrohexitol ; les tétraesters à base de pentaérythritol ; les esters à base de furane et autres esters ; les cétals ; ou les plastifiants polymères.

7. Procédé selon l'une quelconque des revendications 5 à 6, dans lequel lesdites charges comprennent le carbonate de calcium, le carbonate de magnésium, la silice, l'argile, le mica, le graphite, l'oxyde de zinc, ou l'oxyde de calcium ; ou lesdites charges comprennent le carbonate de calcium.

8. Procédé selon l'une quelconque des revendications 5 à 7, dans lequel lesdits additifs de régulation de rhéologie comprennent les distillats de pétrole ; l'huile minérale et les essences minérales ; les esters d'acides gras ; les oligomères de polyphényle ; ou les solvants organiques.

9. Procédé selon l'une quelconque des revendications 1 à 8, dans lequel ledit plastisol fondu a un Tan Delta maximal (Tan δₘₐₓ) qui apparaît entre 20 et 50 °C et un Tan Delta à 30 °C (Tan δ_{30C}) dans la plage de 0,5 à 2,0, mesurés sur un échantillon ayant une épaisseur nominale de 0,6-0,7 mm, une largeur nominale de 3,2 mm, et longueur nominale de 10-12 mm à l'aide d'un analyseur mécanique dynamique avec raccordement à ressort à une contrainte de 0,1 % et fréquence de 1 Hz et vitesse de rampe de température de 3 °C/min.

10. Procédé selon la revendication 9, dans lequel ledit Tan Delta maximal (Tan δₘₐₓ) apparaît entre 20 et 40 °C.

11. Procédé selon l'une quelconque des revendications 1 à 10, dans lequel ladite apposition comprend
a. l'application dudit plastisol sur ledit substrat, où ladite application de plastisol sur ledit substrat comprend le revêtement dudit substrat avec ledit plastisol, et où ledit revêtement comprend le revêtement par pulvérisation et/ou le revêtement par extrusion ;
b. la fusion dudit plastisol pour obtenir un substrat recouvert de plastisol ; et
c. le refroidissement dudit substrat recouvert de plastisol jusqu'aux températures ambiantes.

12. Procédé selon l'une quelconque des revendications 1 à 11, dans lequel ladite apposition comprend
a. la fusion dudit plastisol pour obtenir une feuille ; et
b. le collage de ladite feuille audit substrat.

13. Procédé selon l'une quelconque des revendications 1 à 12, dans lequel ledit substrat fait partie d'un véhicule à roues.
